# EUROPÄISCHE PATENTSCHRIFT

(11) **EP 1 495 115 B1**
(45) Veröffentlichungstag und Bekanntmachung des Hinweises auf die Patenterteilung: **05.10.2011**
(21) Anmeldenummer: 03714857.4
(22) Anmeldetag: 19.03.2003
(51) Int. Cl.: C12N 9/04, C07K 14/37, A61K 39/35

(54) **NUCLEINSÄURESEQUENZ UND PROTEIN SOWIE POLYPEPTIDE KODIEREND FÜR MANNIT-DEHYDROGENASEN ODER DEREN TEILE SOWIE DEREN HERSTELLUNG UND VERWENDUNG IN DIAGNOSTIK UND THERAPIE**
NUCLEIC ACID SEQUENCE AND PROTEIN IN ADDITION TO POLYPEPTIDES CODING FOR MANNITOL-DEHYDROGENASES OR PARTS THEREOF AND THE PRODUCTION AND USE THEREOF IN DIAGNOSIS AND THERAPY
SEQUENCE D'ACIDES NUCLEIQUES ET PROTEINE AINSI QUE POLYPEPTIDES CODANT DES MANNITOL DESHYDROGENASES OU LEURS PARTIES AINSI QUE LEUR PRODUCTION ET LEUR UTILISATION A DES FINS DE DIAGNOSTIC ET DE TRAITEMENT

(30) Priorität: 28.03.2002 DE 10214082; 24.07.2002 DE 10233676
(43) Veröffentlichungstag der Anmeldung: 12.01.2005
(73) Patentinhaber: BIOMAY AG, 1090 Wien (AT)
(72) Erfinder: SIMON-NOBBE, Birgit, A-5020 Salzburg (AT); SCHNEIDER, Peter, A-4894 Oberhofen (AT); DENK, Ursula, A-4060 Leonding (AT); WALLY, Verena, A-5020 Salzburg (AT); RICHTER, Klaus, A-5020 Salzburg (AT); RADAUER, Christian, A-1150 Wien (AT); TEIGE, Markus, A-1040 Wien (AT); EBNER, Christof, A-2345 Brunn am Gebirge (AT); BREITENBACH, Michael, A-5020 Salzburg (AT)
(74) Vertreter: Keller, Günter
(86) Internationale Anmeldenummer: PCT/EP2003/002873
(87) Internationale Veröffentlichungsnummer: WO 2003/083098

(56) Entgegenhaltungen:
- DATABASE SWISSPROT [Online] PIKE M ET AL: "Cladosporium fulvum, NADP-dependent maqnnitol dehydrogenase" retrieved from TREMBL Database accession no. Q96W29 XP002244008
- NOELDNER P K-M ET AL: "Purification and characterization of mannitol dehydrogenase from the fungal tomato pathogen Cladosporium fulvum (syn. Fulvia fulva)." PHYSIOLOGICAL AND MOLECULAR PLANT PATHOLOGY, Bd. 45, Nr. 4, 1994, Seiten 281-289, XP009012224 ISSN: 0885-5765
- HULT K ET AL: "THE DISTRIBUTION OF THE NADPH REGENERATING MANNITOL CYCLE AMONG FUNGAL SPECIES" ARCHIVES OF MICROBIOLOGY, Bd. 128, Nr. 2, 1980, Seiten 253-255, XP009018332 ISSN: 0302-8933
- VOUGE DE M W ET AL: "MOLECULAR CLONING OF IGE-BINDING FRAGMENTS OF ALTERNARI ALTERNATA ALLERGENS" INTERNATIONAL ARCHIVES OF ALLERGY AND IMMUNOLOGY, XX, XX, Bd. 116, Nr. 4, August 1998 (1998-08), Seiten 261-268, XP009012210 ISSN: 1018-2438
- BREITENBACH MICHAEL ET AL: "Enolases are highly conserved fungal allergens." INTERNATIONAL ARCHIVES OF ALLERGY AND IMMUNOLOGY, Bd. 113, Nr. 1-3, 1997, Seiten 114-117, XP009017515 ISSN: 1018-2438

## Beschreibung

Die verschiedensten Stoffe rufen bei bestimmten Personen allergische Reaktionen hervor. Bekannt sind nicht nur Allergien gegen Bestandteile von Tieren, wie beispielsweise Katzenhaare, sondern auch Allergien gegen Pflanzen bzw. Pflanzenteile, wie Blütenpollen. Bekannt sind aber auch Allergien gegen Mikroorganismen, wie beispielsweise Schimmelpilze.

Noeldner et al. [Physiological and Molecular Plant Pathology (1994) S. 281-289] beschreibt die Reinigung und Charakterisierung einer Mannit dehydrogenase aus dem für Tomaten pathogenen Pilz Cladosporium fulvum. Aufgabe dieser Arbeit war es offensichtlich, den Mechanismus aufzuklären, über den Pflanzen pathogene Pilze Kohlenstoff von Ihren Wirtszellen erhalten und verwerten.

Hult et al. [Auch. Microbiol. 128 (1980) S. 253-255] beschreibt die Verteilung des NADPH erzeugenden Mannit-Zyklus unter verschiedenen Pilzspezies. Hierbei handelt es sich um eine Arbeit aus der Grundlagenforschung.

Diesem Stand der Technik sind oben nicht irgendwelche Hinweise zu entnehmen, wonach die beanspruchten Polypeptide eine wesentliche Rolle bei allergischen Erkrankungen spielen können.

Gegenstand der vorliegenden Erfindung ist ein Impfstoff zur Desensibilisierung von Patienten gegen eine Schimmelpilzallergie sowie die Verwendung des Hauptallergens des Schimmelpilzes Cladosporium herbarum in Diagnostik und Therapie. Im Rahmen der vorliegenden Erfindung wurde festgestellt, daß es sich bei diesem Hauptallergen überraschenderweise um eine Mannit-Dehydrogenase (MtDH) handelt. Ein weiterer Gegenstand der vorliegenden Erfindung bezieht sich auf ein Hauptallergen von Alternaria alternata.

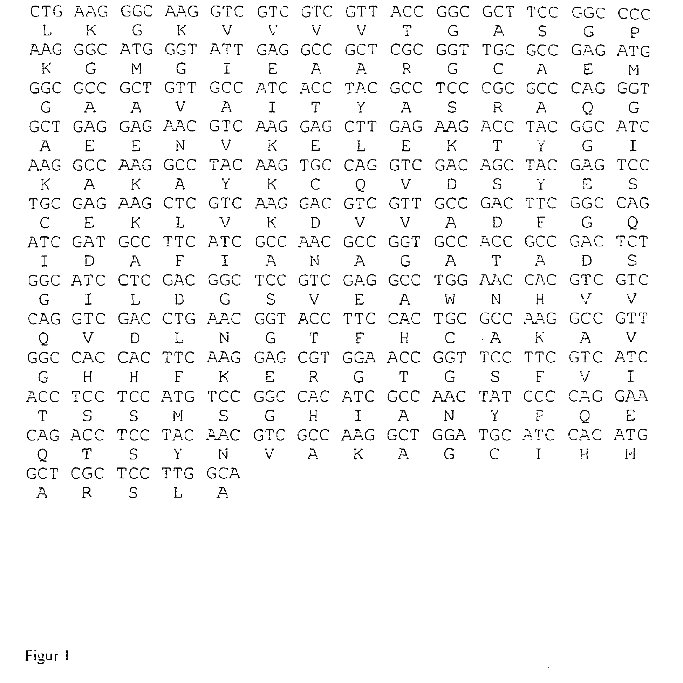

Offenbart wird ein Polypeptid, das wenigstens 10 aufeinanderfolgende Aminosäuren aus der Aminosäuresequenz mit der Sequenz ID Nr. 1 aufweist. Die Aminosäuresequenz ist in Fig. 1 dargestellt, ebenso wie die zugehörige DNA-Sequenz. Die Erfindung betrifft auch die Polypeptide mit den Seq.ID Nr. 4, 5 und 6.

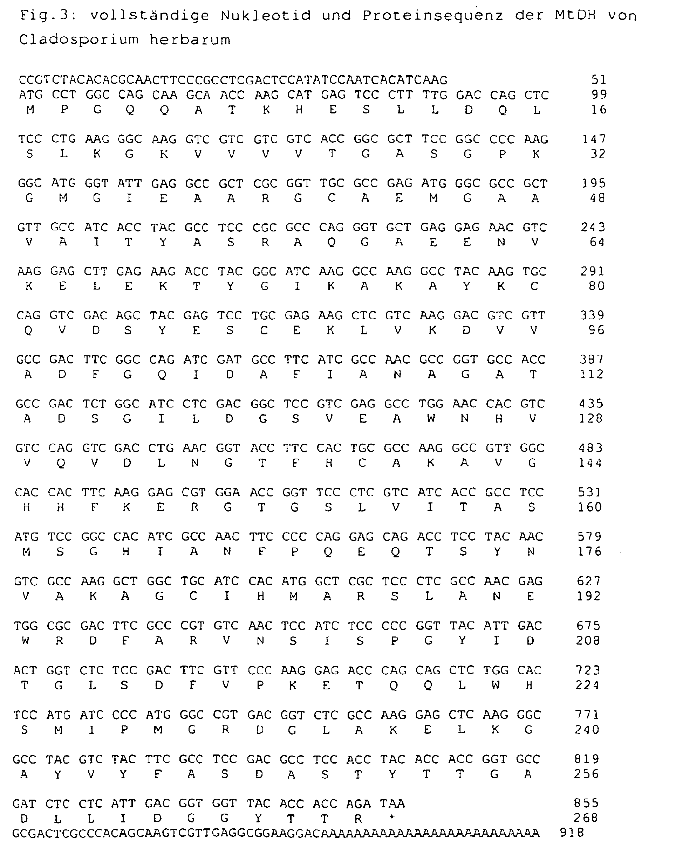

Offenbart wird weiterhin die vollständige Sequenz eines Polypeptids kodierend für eine Mannit-Dehydrogenase. Seq.ID Nr. 7 stellt die Nucleinsäuresequenz und Seq.ID Nr. 8 die Aminosäuresequenz hiervon dar. Die Sequenzen sind in Figur 3 dargestellt.

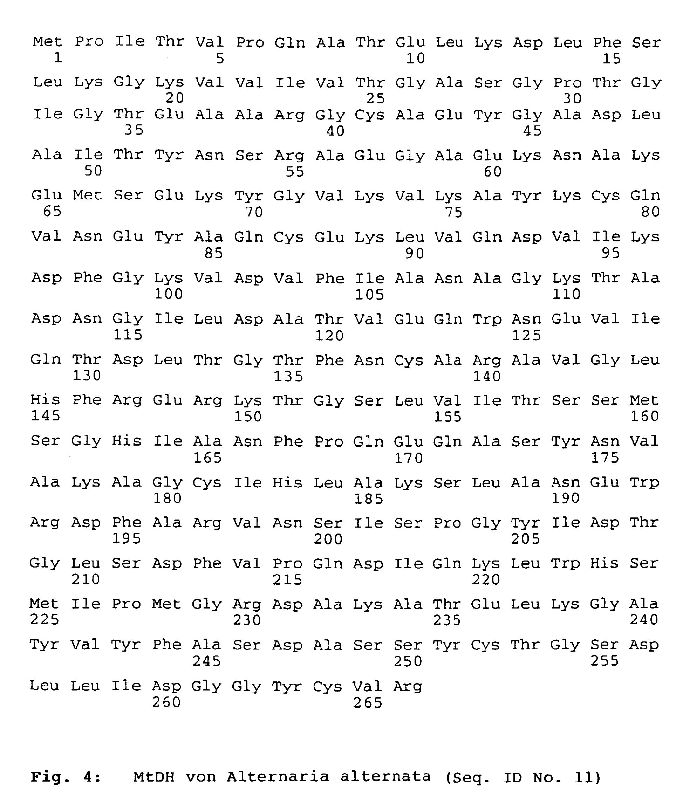

Außerdem wird offenbart ein Polypeptid, das wenigstens 10 aufeinanderfolgende Aminosäuren aus der Aminosäuresequenz mit der Sequenz ID Nr. 11 aufweist. Die Aminosäuresequenz ist in Fig. 4 dargestellt, sie stellt ein Hauptallergen dar, das aus Alternaria alternata isoliert wurde.

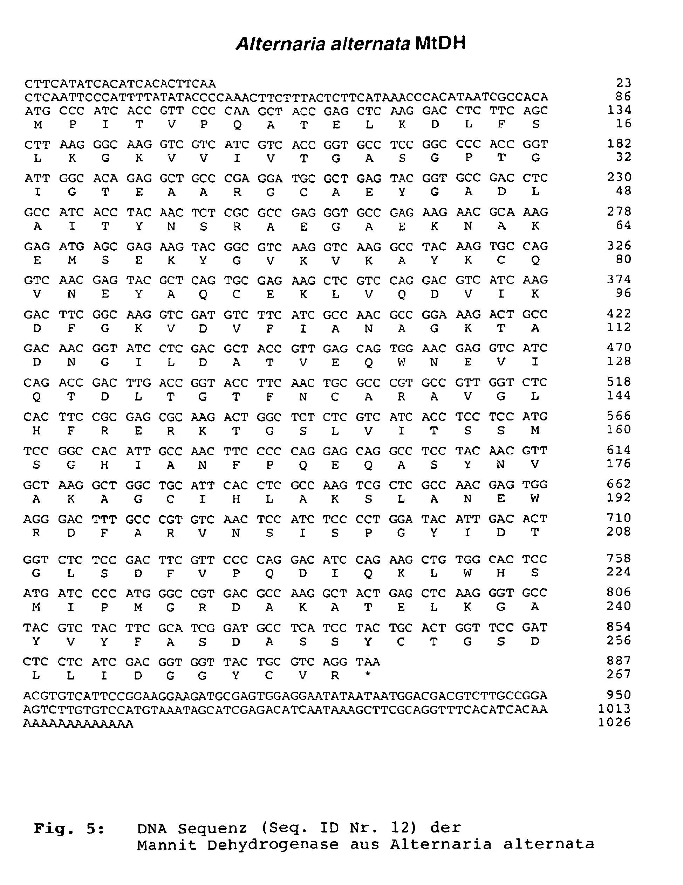

Die Aminosäuresequenz des Hauptallergens aus Alternaria alternata, bei dem es sich um die Mannit-Dehydrogenase handelt, ist im Einbuchstabencode in Fig. 5 wiedergegeben, zusammen mit der hierfür kodierenden DNA-Sequenz (Sequenz ID Nr. 12) sowie den flankierenden Nukleotidsequenzen am 5'- und 3'-Ende.

Ein Polypeptid weist wenigstens ein Epitop auf. Unter einem Epitop versteht man einen Bereich, an den Antikörper binden können. Grundsätzlich gibt es lineare Epitope. In diesem Fall liegen die das Epitop bildenden Aminosäuren nebeneinander. Häufiger sind aber die sogenannten Konformationsepitope. Diese werden durch die Faltung des Polypeptids gebildet. Dabei können Aminosäuren, die in der Sequenz nicht benachbart sind, aufgrund der dreidimensionalen Faltung des Polypeptids in eine räumliche Nähe kommen und diese Oberflächenstruktur wird von einem Antikörper gebunden.

Bevorzugt sind die Epitope spezifisch für einen Schimmelpilz. Grundsätzlich können mit Hilfe geeigneter Techniken, beispielsweise unter Verwendung von Adjuvanzien, Antikörper gegen fast alle Aminosäuresequenzen erzeugt werden. Die erfindungsgemäßen Polypeptide spielen aber bei der Diagnostik und Therapie eine wesentliche Rolle. Deshalb weisen die Polypeptide bevorzugt spezifische Epitope auf, wobei die Epitope spezifisch für einen Schimmelpilz sind. Häufig weisen Proteine bzw. Polypeptide, die aus einem bestimmten Organismus stammen, Ähnlichkeiten auf zu einem entsprechenden Protein bzw. Polypeptid, das von einem verwandten Organismus stammt. Es ist daher möglich, daß Antikörper, die gegen ein Epitop eines bestimmten Schimmelpilzes gerichtet sind, auch mit einem entsprechenden Epitop eines verwandten Schimmelpilzes reagieren.

Je spezifischer ein Epitop ist, desto weniger reagieren hiergegen gerichtete Antikörper mit einem Epitop eines homologen Polypeptids aus einem verwandten Organismus. Die Polypeptide weisen daher bevorzugt solche Epitope auf, die spezifisch sind für einen Schimmelpilz. Besonders bevorzugt weisen die Polypeptide solche Epitope auf, die spezifisch sind für einen Schimmelpilz aus der Gattung Cladosporium. Ganz besonders bevorzugt weisen die Polypeptide ein Epitop auf, das spezifisch ist für Cladosporium herbarum. Antikörper, die gegen ein derartiges Epitop gerichtet sind, reagieren nicht mit anderen Polypeptiden.

Im Rahmen der vorliegenden Erfindung wurde herausgefunden, daß das Polypeptid mit der Aminosäuresequenz ID Nr. 1 für eine Mannit-Dehydrogenase kodiert. Gegenstand der vorliegenden Erfindung sind Impfstoffe mit Teilen dieser Aminosäuresequenz mit wenigstens 11 Aminosäuren. Die erfindungsgemäßen Polypeptide weisen bevorzugt wenigstens 20 aufeinanderfolgende Aminosäuren aus der Sequenz mit der Sequenz ID Nr.

Bevorzugter sind solche Polypeptide, die wenigstens 50 aufeinanderfolgende Aminosäuren aufweisen, und ganz besonders bevorzugt sind solche Polypeptide, die wenigstens 100 aufeinanderfolgende Aminosäuren aus der Aminosäuresequenz mit der Sequenz ID Nr. 1 aufweisen.

Die Erfindung betrifft auch einen Impfstoff mit Polypeptid vom N-Terminus mit der Sequenz PGQQATKHESLLDQLS (Seq.ID Nr. 4) sowie zwei Polypeptide vom C-terminalen Ende mit der Sequenz LDTGLSDFWK (Seq.ID Nr. 5) und MGRDGLAKEL (Seq.ID Nr. 6).

Gegenstand der Erfindung ist auch ein Impfstoff mit einem Polypeptid mit der Sequenz ID Nr. 8 sowie Teile dieses Polypeptids, die ein Epitop enthalten. Die Teile der Sequenz ID Nr. 8 weisen wenigstens 11, bevorzugt wenigstens 20, noch bevorzugter wenigstens 50 und ganz besonders bevorzugt wenigstens 100 aufeinanderfolgende Aminosäuren aus der Aminosäuresequenz mit der Sequenz ID Nr. 8 auf.

Ein weiterer Gegenstand der vorliegenden Erfindung betrifft einen Impfstoff mit einem Polypeptid mit der Sequenz ID Nr. 11 sowie Teile dieses Polypeptids, die ein Epitop enthalten. Derartige Epitope sind spezifisch für Alternaria, genauer für Alternaria alternata. Die Teile der Sequenz ID Nr. 11 weisen wenigstens 11, bevorzugt wenigstens 20, noch bevorzugter wenigstens 50 und ganz bevorzugt wenigstens 100 aufeinanderfolgende Aminosäuren aus der Aminosäuresequenz mit der Sequenz ID Nr. 11 auf.

Bevorzugt weisen diese Polypeptide wenigstens ein Epitop auf. Es ist beispielsweise möglich mit Hilfe von Hydrophilie/Hydrophobie Bestimmungen nachzuweisen, welche Teile des Polypeptids besonders geeignet sind für immunologische Reaktionen. Dies kann beispielsweise mit Hilfe geeigneter Computerprogramme erfolgen.

Alternativ hierzu ist es auch möglich, mit Hilfe der sogenannten Pepscan-Methode Fragmente der Sequenz herzustellen und durch Reaktion mit Seren von allergischen Patienten zu testen, ob die kurzen Fragmente relevante Epitope enthalten. Weiterhin muss geklärt werden, ob es sich hierbei um Epitope handelt, die spezifisch sind für einen Schimmelpilz, insbesondere für einen Schimmelpilz aus der Gattung Cladosporium und/oder Alternaria und insbesondere für Cladosporium herbarum und/oder Alternaria alternata. Zu der Bestimmung bedient man sich der Hilfe geeigneter Serum Panels.

Cladosporium ist eine Pilzgattung, die zu den Schimmelpilzen gehört. Cladosporium-Arten sind sehr häufig und kommen bevorzugt in Sumpfgebieten, im Wald und in Gärten vor, da sie gerne auf verfaulten Pflanzen bzw. auf Laub wachsen. Außerdem trifft man sie in Gewächshäusern und in schlecht gereinigten Kühlschränken an. Auch auf Textilien, z.B. Leinenstoffen, wächst Cladosporium. Cladosporium kann allergische Reaktionen wie z.B. Fließschnupfen, Husten, Niesanfälle, Nesselfieber oder Asthma auslösen (Schimmelpilzallergie).

Alternaria ist eine Pilzgattung, die auch zu den Schimmelpilzen gehört. Alternaria-Arten kommen bevorzugt in Sumpfgebieten, im Wald und in Gärten vor, da sie gerne auf verfaulten Pflanzen bzw. auf Laub wachsen. Im Haushalt findet man sie vor allem auf Mehl, Obst und Gemüse. Sie wachsen aber auch auf verschiedenen Textilien, z.B. Leinenstoffen. Alternaria kann allergische Reaktionen wie z.B. Fließschnupfen, Husten, Niesanfälle, Nesselfieber oder Asthma auslösen (Schimmelpilzallergie).

Durch die Offenbarung der Aminosäure- und Nucleinsäuresequenz ist es möglich, mit Hilfe rekombinanter Techniken kürzere Fragmente der vollständigen Sequenz rekombinant in Bakterien, beispielsweise in E. coli oder in höheren Organismen, beispielsweise Insektenzellen, Hefen oder eukoryontischen Zellen herzustellen. Gerade kurze Polypeptide können auch gut chemisch mit Hilfe der Festphasensynthese bereitgestellt werden.

Der erfindungsgemäße Impfstoffkann zur Desensibilisierung von Patienten gegen eine Schimmelpilzallergie eingesetzt werden. Bei der Desensibilisierung werden Patienten, die an einer Allergie leiden, mit einer geringen Menge eines Antigens in Kontakt gebracht, wodurch neutralisierende IgE-Antikörper gebildet werden sollen. Durch diese neutralisierenden Antikörper werden die Antigene, mit denen der Patient in Kontakt gekommen ist, gebunden. Dadurch wird die Antigen-Antikörper-Bindung von allergische Reaktionen auslösenden Antikörpern von IgE-Typ vermieden. Die erfindungsgemäßen Polypeptide können daher zur Herstellung eines Impfstoffes verwendet werden. Hierzu können die rekombinant oder auch chemisch hergestellten Polypeptide in eine geeignete Impfstofformulierung eingearbeitet werden. Die Impfstofformulierung kann neben den Polypeptiden auch übliche Zusatzstoffe und Formulierungshilfen, wie auch Adjuvanzien enthalten.

Ein anderer Gegenstand der vorliegenden Erfindung ist die Verwendung eines Polypeptids zu einem diagnostischen Nachweis einer Erkrankung. Bei einer derartigen Erkrankung handelt es sich üblicherweise um eine Allergie. Die Polypeptide werden in einem geeigneten diagnostischen Nachweissystem eingesetzt. Hierbei kann es sich um einen Radioimmunassay (RIA) handeln, oder bevorzugt auch um einen ELISA (Enzyme Linked Immunosorbent Assay). Die übliche Konfiguration eines derartigen diagnostischen Tests ist bekannt.

Offenbart wurden auch Nukleotide aus der Nukleotidsequenz mit der Sequenz ID Nr. 2. Die Nukleotidsequenz mit der Sequenz ID Nr. 2 ist ebenfalls in Figur 1 dargestellt. Es handelt sich hierbei um einen Teil des Gens für die erfindungsgemäße Mannit-Dehydrogenase aus Cladosporium herbarum.

Beschrieben werden weiterhin Polynukleotide mit der Nukleotidsequenz der Sequenz ID Nr. 7 sowie Teile dieser Nukleotidsequenz. Mit Hilfe dieser Nucleotidsequenz oder Teilen davon kann ein gewünschtes Polypeptid rekombinant in geeigneten Wirtszellen hergestellt werden.

Offenbart werden Polynukleotide mit der Nukleotidsequenz, Sequenz ID Nr. 12. Hierbei handelt es sich um eine Nukleotidsequenz, die für die Mannit-Dehydrogenase aus Alternaria alternata kodiert sowie Nukleotidsequenzen, die dem kodierenden Bereich unmittelbar benachbart sind. Auch Teile dieser Nukleotidsequenz werden beschrieben.

Ein Polynukleotid weist wenigstens acht aufeinanderfolgende Nukleotide, bevorzugt wenigstens 12, noch bevorzugter wenigstens 20 und am bevorzugtesten wenigstens 50 aufeinanderfolgende Nukleotide auf. Für manche Einsatzgebiete müssen die Nukleotide länger sein, in diesem Fall weisen die Polynukleotide wenigstens 100 aufeinanderfolgende Nukleotide ausgewählt aus der Sequenz ID Nr. 2, ID Nr. 7 oder Sequenz ID Nr. 12 auf.

Die Polynukleotide können zum Nachweis einer Mannit-Dehydrogenase dienen. Bevorzugt wird dabei das Vorhandensein eines Gens kodierend für diese Mannit-Dehydrogenase aus Cladosporium herbarum und/oder Alternaria alternata nachgewiesen. Bei diesen Verfahren handelt es sich um an sich bekannte Nukleinsäureamplifikationsmethoden. Hierfür kommt beispielsweise die NASBA (nucleic acid sequence based amplification) in Frage oder bevorzugter die Polymerase Kettenreaktion (PCR).

Da die Nucleinsäuresequenzen kodierend für die Mannit-Dehydrogenase aus Cladosporium herbarum und Alternaria alternata offenbart werden, ist es möglich, solche Nukleotidsequenzen für die Amplifikation zu wählen, die eine sehr hohe Homologie oder sogar Identität aufweisen. Es kann erwartet werden, dass bei Verwendung derart hochspezifischer Primer eine Amplifikation auch von anderen Mannit-Dehydrogenasen aus verwandten Organismen erfolgt, weil eine hohe Homologie bei den Aminosäuresequenzen für eine starke Konservierung in einem solchen Genbereich spricht.

Für die Nucleinsäurediagnostik ist es daher bevorzugt, solche Bereiche, die stark konserviert sind, dann zu verwenden, wenn das Antigen nicht nur aus Cladosporium und/oder Alternaria-Spezies, sondern auch aus anderen Schimmelpilzspezies isoliert werden soll.

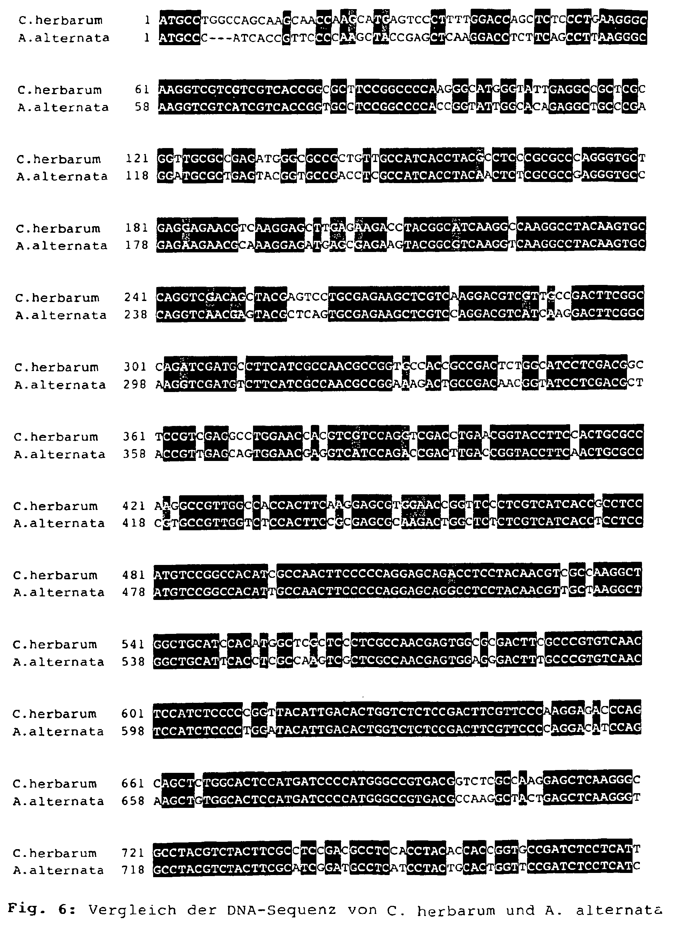

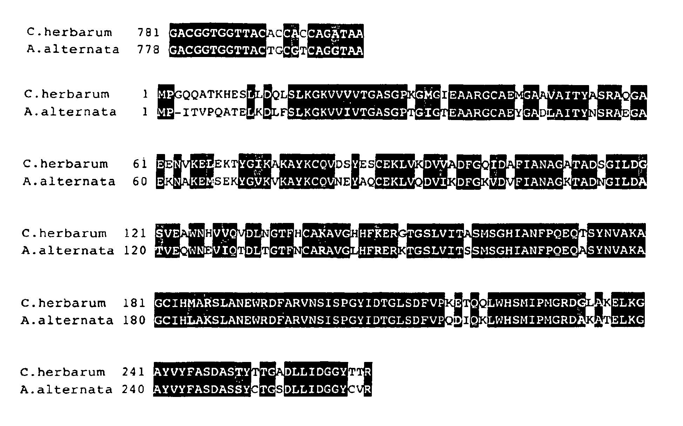

Bereiche, die eine geringe Homologie zueinander aufweisen, eignen sich daher besser für die Feindiagnostik, also für die Unterscheidung sowohl zwischen Alternaria wie auch Cladosporium-Spezies, wie auch für die Feindifferenzierung innerhalb von Cladosporien oder Alternarien. In Fig. 6 sind die kodierenden Bereiche aus Cladosporium herbarum und Alternaria alternata zusammen dargestellt. Identische Nukleotidsequenzen sind schwarz hinterlegt und weisen auf konservierte Bereiche hin.

Mit Hilfe eines derartigen Verfahrens kann das Vorhandensein des Schimmelpilzes Cladosporium herbarum und/oder Alternaria alternata nachgewiesen werden. Derartige Anwendungen sind nicht nur in der medizinischen Diagnostik von Interesse, sondern auch in anderen Bereichen, beispielsweise der Hygiene und der Untersuchung von Lebensmitteln. Berücksichtigt werden muß hier, daß Cladosporium herbarum auch bei verhältnismäßig niederen Temperaturen bis zu etwa +6C° wachsen kann und daher in Bereichen der Lebensmitteltechnologie eine unerwünschte Kontamination darstellen kann. Für die Überwachung von Lebensmitteln und deren Qualitätskontrolle kann der Nachweis auch von geringen Mengen von Cladosporium herbarum eine wesentliche Rolle spielen.

In einem weiteren Aspekt wird ein Verfahren zur Herstellung eines Polypeptids offenbart. Zunächst kann mit Hilfe der Polynukleotide und mit Hilfe der Polymerase Kettenreaktion ein Gen aus einem Schimmelpilz, bevorzugt aus Cladosporium herbarum oder Alternaria alternata, amplifiziert werden. Dieses Polynukleotid kann dann in einen geeigneten Vektor eingebaut werden, mit dem eine Wirtszelle transformiert wird. Geeignete Vektoren vermehren sich in der Wirtszelle und dabei werden die Polypeptide exprimiert. Bei den Wirtszellen kann es sich um übliche Wirtszellen handeln. In Frage kommen hierbei bakterielle Wirtszellen wie Escherichia coli oder Bacillus subtilis oder Hefen wie beispielsweise Saccharomyces cerevisiae oder Pichia pastoris.

Im Rahmen der vorliegenden Erfindung wurden IgE Immunblots von Rohextrakt aus Cladosporium herbarum mit 62 Patientenseren getestet. Hierbei zeigte sich ein immunreaktives Protein vom Molekulargewicht 29 kD, das von 61 % der Patientenseren erkannt wurde. Die Patienten waren im Hauttest bzw. Bluttest (RAST) vorselektioniert und reagierten positiv auf Cladosporium herbarum Extrakt. Kein anderes Allergen im Extrakt aus Cladosporium herbarum reagiert mit einer so großen Prozentzahl an Patientenseren. Es wird daher davon ausgegangen, daß dieses Protein das Hauptallergen von Cladosporium herbarum ist.

Die im Rahmen der vorliegenden Erfindung offenbarten immunreaktiven Proteine stellen wichtige Allergene nicht nur für die Diagnose, sondern auch für die Therapie von Allergen gegen Schimmelpilze, insbesondere Cladosporium- und Alternaria-Spezies dar. Gegebenenfalls können diese Allergene zusammen mit anderen Allergenen, beispielsweise dem Alt a 1 [Unger A. et al. (1999), Clinical testing of recombinant allergens of the mold Alternaria alternata, Int. Arch. Allergy Immunol. 118, 220-221] und Enolase [Simon-Nobbe B. et al. (2000), IgE binding epitopes of enolases, a class of highly conserved fungal allergens, I. Allergy Clin. Immunol. 106, 887-895] sowohl bei der Diagnostik wie auch bei der Therapie eingesetzt werden.

In der zweidimensionalen Auftrennung durch isoelektrische Fokussierung und SDS-PAGE wurde dieses Protein aus Cladosporium herbarum als ein Spot von 29 kD und dem isoelektrischen Punkt bei pH=5.8 dargestellt.

Das Protein wurde in einem konventionellen Verfahren (Beispiel 1) zur Homogenität gereinigt. Die Ausbeute war 1 mg. Das homogen gereinigte Protein wurde nun im IgE-Immunblot mit einem Pool von sechs Patienten getestet und war stark positiv (Beispiel 2).

Das zur Homogenität gereinigte Protein wurde durch automatisierten Edman-Abbau N-terminal ansequenziert und interne Peptidsequenzen wurden nach CNBr Abbau ermittelt. N-terminale und interne Peptidsequenzen nach Trypsinverdau wurden durch Edman-Abbau von etwa 50 µg Protein ermittelt, die durch Ausschneiden eines Spots aus der zweidimensionalen Elektrophorese gewonnen wurden.

Tabelle 1 zeigt eine Liste aller ermittelten Peptidsequenzen. Es wurde der "single letter code" verwendet. Die in Tabelle 1 schwarz unterlegten Aminosäuren konnten in der Sequenz der Mannit-Dehydrogenase aus Cladosporium fulvum aufgefunden werden.

### Peptidsequenzen der NADP-abhängigen Mannit-Dehydronenase von C. herbarum N-terminale Sequenz

**Tabelle 1**

| | | |
|---|---|---|
| | | a) Ausgangsmaterial: Rohextrakt aufgetrennt mittels 2-dimensionalem SDS-Gel b) Analyseverfahren: Edman Sequenzierung |
| | | a) Ausgangsmaterial: nativ gereinigtes Protein b) Analyseverfahren: Edman Sequenzierung |
| | **Peptid 1** | |
| | a) Ausgangsmaterial: Rohextrakt aufgetrennt mittels 2-dim. SDS-Gel b) Analyseverfahren: MS/MS c) Bemerkung: überlappt mit der N-terminalen Sequenz | |
| | **Peptid 2** | |
| | a) Ausgangsmaterial: Rohextrakt aufgetrennt mittels 2-dim. SDS-Gel b) tryptischer Verdau c) Analyseverfahren: Sequenzierung | |
| | a) Ausgangsmaterial: Rohextrakt aufgetrennt mittels 2-dim. SDS-Gel b) tryptischer Verdau c) Analyseverfahren: MS/MS | |
| | **Peptid 3** | |
| | a) Ausgangsmaterial: Rohextrakt aufgetrennt mittels 2-dim. SDS-Gel b) tryptischer Verdau c) Analyseverfahren: Sequenzierung | |
| | **Peptid 4** | |
| | a) Ausgangsmaterial: Rohextrakt aufgetrennt mittels 2-dim. SDS-Gel b) tryptischer Verdau c) Analyseverfahren: MS/MS | |
| | **Peptid 5** | |
| | a) Ausgangsmaterial: nativ gereinigtes Protein b) CnBr - Verdau c) Analyseverfahren: Sequenzierung | |

Die Peptidsequenzen wurden durch computergestützte Homologiesuche mit allen in den Datenbanken (Swissprot, Genbank u.a.) aufgelisteten Proteinsequenzen verglichen. Alle Peptide zeigten Homologie zur Familie der Mannit-Dehydrogenasen. Die Mannit-Dehydrogenase, zu der die Peptide die höchste Sequenzähnlichkeit aufweisen, ist die Mannit-Dehydrogenase aus Cladosporium fulvum. Eine Reinigung von Mannit-Dehydrogenase aus Cladosporium fulvum ist in Noeldner et al., Physiological and Molecular Plant Pathology (1994) S. 281-289 beschrieben. Die Position dieser Peptide in der Sequenz sind in dem "alignment" (Tabelle 2) sichtbar.

### Protein-Alignment der NADP-MtDH von C. fulvum mit Peptidsequenzen von C. herbarum

**Tabelle 2**

| | | | |
|---|---|---|---|
| C.fulvum C.herbarum | 1 | | |
| C.fulvum C.herbarum | 61 | | |
| C.fulvum C.herbarum | 121 | | |
| C. fulvum C.herbarum | 181 | | |
| C.fulvum C.herbarum | 241 | YVYLVSDASTYTTGADIVIDGGYTCR -------------------------- | |
| C.fulvum MtD H: | | accession number: AAK67169 (Seq.ID Nr. 3) | |
| | | Länge der codierenden Sequenz: | 267 Aminosäuren (AA) |
| | | | 801 Basenpaare (bp) |
| | | MW: 28,6kD | |
| | | pl:6.33 | |

In Tabelle 2 ist die Anordnung der Polypeptide mit den Seq.ID Nr. 4, 5, und 6 anhand der Homologie mit C.fulvum gezeigt.

Aufgrund dieser Ergebnisse wurde die Enzymaktivität des zur Homogenität gereinigten Proteins geprüft. Die Experimente ergeben, dass das hochgereinigte Hauptallergen von Cladosporium herbarum tatsächlich eine Mannit-Dehydrogenase ist, die die folgende Stoffwechselreaktion katalysiert: Fructose + NADPH + H⁺ ⇔ Mannit + NADP⁺. Ferner wurde gefunden, dass NADH als Co-substrat nicht aktiv ist und dass auch Fructose-6-Phosphat als Substrat nicht aktiv ist. Fructose-6-Phosphat wirkt auf die Reaktion als Inhibitor. Die Methode der Aktivitätsbestimmung ist in Beispiel 3 beschrieben.

Es wurden nunmehr die N-terminale und eine interne Peptidsequenz der Mannit-. Dehydrogenase von Cladosporium herbarum benützt, um durch Rückübersetzung PCR-primer zu entwerfen. Die Auswahl der Primer ist in Tabelle 3 zusammengefaßt.

### DNA-Sequenz der von den Peptiden abgeleiteten Oligos

**Tabelle 3**

| |
|---|
| **Oligo 1:** |
| • abgeleitet von der N-terminalen Sequenz der Mannit-Dehydrogenase (MtDH) von C. herbarum (siehe Seq.ID Nr. 2) |
| • Oligosequenz: 5' CA(A/G) CA(A/G) GC(I/C) AC(I/C) AA(A/G) CA(C/T) GA 3' |
| **Oligo 2:** |
| • abgeleitet von Peptid 4 der MtDH von C. herbarum |
| • Oligosequenz: 5' AC(A/G) AA(A/G) TC(A/G) CT(I/C) AG(I/C) CC(A/G) GT(A/G) TC 3' |
| Die Primer sind Gemische von synthetischen Oligonukleotiden. (A/G)...bedeutet, daß an dieser Stelle sowohl Adenin als auch Guanin in den Oligonukleotiden vorkommen. Ebenso bei (C/T) und (I/C), wobei I für die Base Inosin steht. |

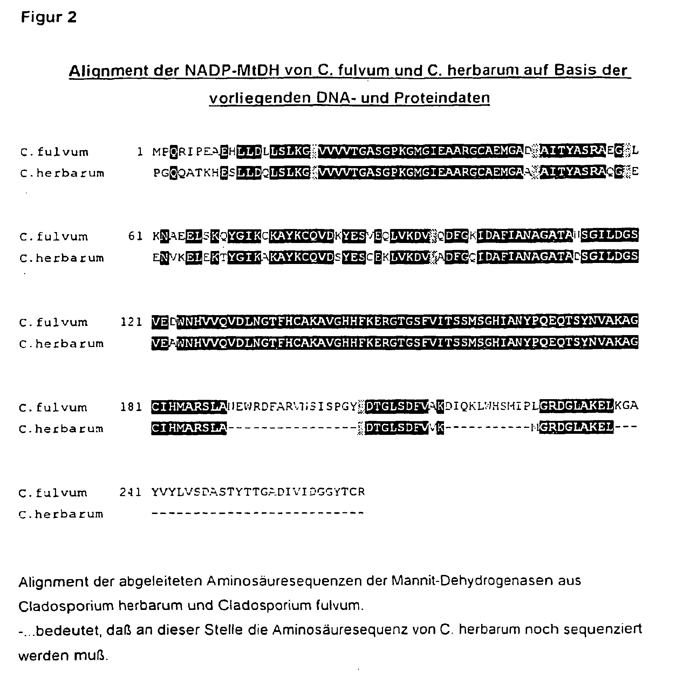

Mit diesen in Tabelle 3 gezeigten Primern wurde eine PCR-Reaktion mit DNA einer von den Erfindern konstruierten cDNA-Bank aus Cladosporium herbarum (Achatz G et al., 1995, Mol. Immunol., 32; 213-27) durchgeführt. Das Ergebnis war eine Bande von 636 bp. Diese wurde durch automatisierte DNA-Sequenzierung nach Sanger (1977, Proc. Natl. Acad. Sci. USA, 74; 5463-7) unter Verwendung der PCR-Primer als Sequenzierungsprimer sequenziert. Dabei wurde Seq.ID Nr. 2 ermittelt. Die von dieser DNA-Sequenz abgeleitete Proteinsequenz (Seq.ID Nr. 1) ist zu 87 % identisch mit der Proteinsequenz der Mannit-Dehydrogenase aus Cladosporium fulvum. Zieht man auch die Substitution durch chemisch verwandte Aminosäuren (zB. I - V, Isoleucin - Valin etc.) in Betracht, so erhöht sich dieser Wert auf 92 %. Unter der plausiblen Annahme, dass die Mannit-Dehydrogenase aus Cladosporium herbarum ebenso wie die Mannit-Dehydrogenase aus Cladosporium fulvum eine Gesamtlänge von 267 Aminosäuren aufweist, wurde durch Peptidsequenzierung einerseits und durch DNA-Sequenzierung andererseits bereits 65 % der Aminosäuresequenz des Hauptallergens (Mannit-Dehydrogenase) aus Cladosporium herbarum bestimmt. Die gesamte bisher bekannte Sequenz dieses Proteins und ihr Alignment mit der homologen Sequenz aus Cladosporium fulvum ist in Figur 2 dargestellt.

### Beispiel 1

### Proteinreinigung

### 1. Ammoniumsulfatfällung:

Durch eine Ammoniumsulfatkonzentration von 50 % kann eine Vorfraktionierung erreicht werden, wobei Mannit-Dehydrogenase (MtDH) im Überstand verbleibt.

Dem Extrakt wurden 50 mM Tris-HCl, pH 7.5 zugesetzt. Proteasen wurden mit 1 Tablette Roche Complete je 100 ml Extrakt und 2 m M EDTA inhibiert.

Die Fällung erfolgte durch Zugabe von festem, gemahlenem Ammoniumsulfat und wurde in zwei Schritten durchgeführt, zuerst 0-30 %, anschließend 30-50 %. Die Fällung wurde für mind. 45 min. äquilibriert, bevor der Extrakt bei 12 000g zentrifugiert wurde. Der Überstand wurde filtriert und über die hydrophobe Interaktionschromatographie (HIC) weitergereinigt.

### 2. HIC (Phenylsepharose):

Der Überstand von der Ammoniumsulfatfällung wurde mit 3 M Natriumacetat auf pH 6.5 eingestellt. Die Säule (8 ml Source 15 PHE, PHARMACIA) wurde mit 1,2 M Ammoniumsulfat, 50 mM Tris-HCl, pH 7.5, 2 mM EDTA äquilibriert und bei einer Durchflußrate von 1 ml/min mit der Probe beladen. Nach Waschen der Säule mit 20 ml Puffer wurde mit einem Gradienten über 40 ml von 1,2 M Ammoniumsulfat bis 0,6 M Ammoniumsufat eluiert.

Die Mannit-Dehydrogenase (MtDH)-Fraktionen wurden gepoolt und für den Anionentauscher vorbereitet. Das Volumen wird über Centricon-Zentrifugenröhrchen (Millipore) eingeengt; Pufferaustausch gegen 50 mM Tris-HCl, pH 7.5 mit Hilfe von PD-10 Desalting Columns (AMERSHAM-PHARMACIA).

### 3. Anionentauscher (Q-Sepharose):

Die Säule (8 ml Source 15 Q, PHARMACIA) wurde mit 15 mM Tris-HCl, pH 7.5 äquilibriert. Eluiert wurde mit einem NaCl-Gradienten von 0-300 mM über 100 ml.

### Beispiel 2

### Immunblot der nativ gereinigten MtDH nach Auftrennung im SDS-Gel

Nativ gereinigte MtDH wurde in einem reduzierenden SDS-Gel (Laemmli UK, Nature, 1970; 27:680-5) nach dem Molekulargewicht aufgetrennt. Das Protein wurde anschließend in einem Western Blot (Towbin H et al., Proc. Natl. Acad. Sci USA, 1979; 76: 4350-4) auf eine PVDF-Membran transferiert. Nach der Absättigung freier Bindungsstellen (30 min in Blockingpuffer: 50 mM Na-phosphat pH 7.5, 0,5 % Tween20, 0.5 % BSA, 0.05 % NaN₃), erfolgte die Inkubation der Membran mit Patientenserum (1:10 verdünnt in Blockingpuffer). Dann wurde die Membran (3x10 min mit Blockingpuffer) zur Entfernung unspezifisch gebundener Antikörper gewaschen. Der Nachweis spezifisch gebundener IgE-Ak erfolgte mit Hilfe eines ¹²⁵J-markierten rabbit-anti-human IgE Antikörpers. Nach Exponierung der Membran auf einem Röntgenfilm konnte das Ergebnis abgelesen werden.

### Ergebnisse:

1) Die nativ gereinigte MtDH reagiert spezifisch mit den IgE-Antikörpern von C. herbarum Allergikern. Es zeigt sich eine prominente IgE-reaktive Bande bei 29kD.
2) Dasselbe Resultat, nämlich eine prominente IgE-reaktive Bande bei 29kD, erhält man, wenn ein C. herbarum Gesamtextrakt im SDS-Gel aufgetrennt und anschließend im Immunblot mit Patientenserum inkubiert wird.

### Beispiel 3

### Immunblot der nativ gereinigten MtDH nach 2 dimensionaler Auftrennung

Nativ gereinigte MtDH wurde unter denaturierenden Bedingungen in einer Isoelektrischen Fokusierung (O'Farrel PH, J. Biol. Chem., 1975; 250: 4007-21) entsprechend der Nettoladung (isoelektrischer Punkt) des Proteins aufgetrennt. Im Anschluß daran wurde das so aufgetrennte Protein einer SDS-Gelelektrophorese (Laemmli UK, Nature, 1970; 27:680-5) unterzogen, wodurch zusätzlich eine Auftrennung nach dem Molekulargewicht erfolgte. Das Protein wurde in einem Western Blot (Towbin H et al., Proc. Natl. Acad. Sci USA, 1979; 76: 4350-4) auf eine PVDF-Membran transferiert. Nach der Absättigung freier Bindungsstellen (30min in Blockingpuffer: 50mM Na-phosphat pH 7.5, 0,5 % Tween 20, 0.5 % BSA, 0.05 % NaN₃), erfolgte die Inkubation der Membran mit Patientenserum (1:10 verdünnt in Blockingpuffer). Anschließend wurde die Membran (3x10 min mit Blockingpuffer) zur Entfernung unspezifisch gebundener Antikörper gewaschen. Der Nachweis spezifisch gebundener IgE-Ak erfolgte mit Hilfe eines ¹²⁵J-markierten rabbit-anti-human IgE Antikörpers. Nach Exponierung der Membran auf einem Röntgenfilm konnten folgende Ergebnisse erkannt werden:

### Ergebnisse:

1) Die nativ gereinigte MtDH reagiert spezifisch mit den IgE-Antikörpern von C. herbarum Allergikern. Beobachtet wurde ein prominenter IgE-reaktiver Spot bei einem Molekulargewicht von 29 kD und einem isoelektrischen Punkt von 5,8.
2) Dasselbe Resultat, nämlich einen prominenten IgE-reaktiven Spot bei einem Molekulargewicht von 29 kD und einem isoelektrischen Punkt von 5.8, erhält man, wenn ein C. herbarum Gesamtextrakt im zweidimensionalen Gel aufgetrennt und anschließend im Immunblot mit Patientenserum inkubiert wird. Zusätzlich findet man im Gesamtextrakt ein IgE-reaktives Protein mit einem Molekulargewicht von 29 kD und einem isoelektrischen Punkt von 5.6. Dieses Protein könnte eine Isoform der MtDH darstellen.

### Beispiel 4

Zum Beleg der erfindungsgemäßen Ergebnisse wurde eine Bestimmung der Enzymaktivität mit dem herkömmlich gereinigten Protein durchgeführt. Die Messung der Absorption von NADPH erfolgte bei 340 nm im Photometer.

### Reaktionsansatz (1 ml):

50 mM Tris-HCl, pH 7.5
0,25 mM NADPH bzw. NADH
D-Fructose bzw. Fructose-6-phosphat (0,1; 0,2; 0,4; 0,6; 0,8; 1,0; 1,2 M)
ad 1 ml H₂O
Start der Reaktion mit 0,5 µl MtDH

### Ergebnisse:

Reaktion mit Fructose und NADPH.
Keine Reaktion mit Fructose-6-Phosphat und NADH

### Beispiel 5

### Sequenz der Mannitdehydrogenase (MtDH)

Die vollständige Sequenz der Mannitdehydrogenase aus Cladosporium herbarum wurde ermittelt wie im folgenden beschrieben.

Die durch Edman Abbau der zur Homogenität gereinigten Mannitdehydrogenase aus Cladosporium erhaltenen Peptidsequenzen wurden benützt, um Primer-Gemische für die PCR zu synthetisieren. PCR ergab eine Bande von 636 nt, die einerseits sequenziert und andererseits als Hybridisierungsprobe zum Screenen unserer cDNA Bank benützt wurde. Es wurde ein vollständiger Klon von Cladosporium herbarum Mannitdehydrogenase (MtDH) isoliert und sequenziert. Die vollständige Sequenz ist in Fig. 3 gezeigt und ist zu 84% identisch mit der publizierten Sequenz der MtDH von C. fulvum.

Tabelle 4 zeigt das Sequenzalignment der beiden Mannitdehydrogenasen von Cladosporium herbarum und Cladosporium fulvum, wobei nur die unterschiedlichen Aminosäuren dargestellt sind.

**Tab. 4: Vergleich der Aminosäuresequenzen der MtDHs von C. herbarum und C. fulvum:**

| | | |
|---|---|---|
| C.fulvum C.herbarum | 1 1 | |
| C.fulvum C.herbarum | 60 61 | |
| C.fulvum C.herbarum | 120 121 | |
| C. fulvum C.herbarum | 180 181 | |
| C.fulvum C.herbarum | 240 241 | |

Schwarz unterlegt bedeutet identische Aminosäuresequenz, grau unterlegt sind chemische ähnliche Aminosäuren und unterschiedliche Aminosäuren sind ohne Unterlegung dargestellt.

Die Sequenz von C. fulvum ist als Seq ID NO:9 und die Aminosäuresequenz von C. herbarum ist als SEQ ID NO: 10 wiedergegeben.

Aus Tabelle 4 ist ersichtlich, welche Bereiche des Polypeptids sich eventuell für den Nachweis oder einen Impfstoff für Cladosporium eignen. Hierbei handelt es sich um die Bereiche, die keine Unterschiede aufweisen.

Bei den Bereichen, die starke Unterschiede aufweisen, ist davon auszugehen, dass die immunologischen Reaktionen unterschiedlich ausfallen, daher können solche Bereiche hochspezifische Epitope beinhalten.

Bei der Ermittlung der in Fig. 3 wiedergegebenen Sequenz wurde festgestellt, dass geringfügige Unterschiede in der Nukleotidsequenz, verglichen mit den anfangs isolierten Teilsequenzen auftraten. Dies kann auf unterschiedliche Sequenzen zurückzuführen sein, die sich in der Genbank befanden. Diese Unterschiede ändern aber nichts an der vorliegenden Erfindung. Gegenstand der Erfindung sind die offenbarten unterschiedlichen Sequenzen, da davon ausgegangen wird, dass es sich hierbei um Varianten des Gens handelt.

### Beispiel 6

### Expression in E. coli und Reaktivität mit Patientenserum.

Der offene Leserahmen der MtDH wurde in folgende Expressionsvektoren hineinkloniert:
a) pHis-Parallel 2 Vektor (XhoI/BamHI) (Ref.: P. Sheffield, S. Garrard, and Z. Derewenda (1999). Overcoming expression and purification problems of RhoGDI using a family of "parallel" expression vectors. Protein Expr Purif 15, 34.)
b) pMW172 Vektor (NdeI/EcoRI) (Ref.: M. Susani, P. Jertschin, C. Dolecek, W. R. Sperr, P. Valent, C. Ebner, D. Kraft, R. Valenta, and O. Scheiner (1995). High level expression of birch pollen profilin (Bet v 2) in Escherichia coli: purification and characterization of the recombinant allergen. Biochem Biophys Res Commun 215, 250.)

Die Plasmide wurden anschließend in den *Escherichia coli* Stamm BL21 (DE3) transformiert. Für die darauffolgende Induktion wurden je 5ml LBamp mit 50µl einer stationären Übernachtkultur der zwei Klone angeimpft und bei 37°C solange geschüttelt, bis ein OD₆₀₀ von 0,8 erreicht war (ca. 4h). Die Induktion der Proteinexpression erfolgte mit 0,8 mM IPTG. Nach einer 4-stündigen Inkubation bei 37°C im Schüttelinkubator wurden die *E*. *coli-*Suspensionen 15 min. bei 4000 rpm abzentrifugiert. Die Bakterienpellets wurden anschließend in 1ml 1xPBS resuspendiert und je 6µl der gelösten Bakterienpellets mittels SDS-PAGE aufgetrennt und anschließend mit Coomassie BBR250 gefärbt. Dabei wurden folgende Ergebnisse erhalten: Die mit den Expressionsplasmiden transformierten und mit IPTG induzierten E, coli Zellen, nicht aber die E. coli Zellen ohne Plasmid zeigen eine starke Proteinbande beim jeweils erwarteten Molekulargewicht, nämlich 30 kD, bzw. etwa 33 kD (apparent molecular weight).

Mit den mit Hilfe eines Gels aufgetrennten Polypeptiden wurde ein IgE-Immunblot durchgeführt. Verwendet wurde Serum eines Cladosporium-positiven Allergikers. Der Nachweis der gebundenen IgE-Antikörper erfolgte mit dem ¹²⁵Jod markierten rabbit-anti-human-IgE-Antikörper (RAST). Die beiden in E. coli überexprimierten Fremdproteine reagieren stark mit dem IgE des Patienten, nicht aber die Proteine von E. coli selbst.

### Beispiel 7

### Ermittlung der Häufigkeit der Reaktion gegen rekombinante MtDH mit Hilfe von 30 Seren von Cladosporium-positiven Allergikern.

Der in Beispiel 6 beschriebene Versuch wurde wiederholt, wobei aber 30 verschiedene Cladosporium-positive nicht vorselektierte Allergikerseren verwendet wurden. Die Kontrolle zeigte eine sehr schwache Immunreaktivität des E. coli Extraktes mit dem zweiten Antikörper (RAST). Dies ist vermutlich auf ein Artefakt zurückzuführen. Andere Kontrollen waren wie erwartet negativ.

Von 30 Patienten zeigten 20 eine IgE-positive Bande bei 30 kD die stärker war als die schwache Bande im Kontrollversuch. Somit wird MtDH von etwa zwei Drittel der Cladosporium-positiven Allergiker erkannt. Dieser Befund ist wichtig, weil durch diesen Versuch gezeigt wird, dass rekombinante Cladosporium herbarum MtDH als Diagnostikum und Therapeutikum für einen Großteil der Patienten einsetzbar ist.

### Beispiel 8

Für die Klonierung der Mannit-Dehydrogenase aus Alternaria alternata wurde eine cDNA Bank in Lambda-ZAP (Stratagene, La Jolla, CA, USA). Diese cDNA Klonbank wurde mit Hilfe von isolierter mRNA aus Alternaria alternata hergestellt.

Wie vorstehend beschrieben, wurde die cDNA Bank mit einer DNA-Sonde gescreent und dabei wurden zunächst 24 Primärklone isoliert. 5 dieser Klone wurden vollständig sequenziert. Im kodierenden Bereich waren alle 5 Sequenzen identisch. Die Übersetzung der Nukleotidsequenz in eine Aminosäuresequenz und der Vergleich mit der Aminosäuresequenz der Mannit-Dehydrogenase aus Cladosporium herbarum zeigte, dass der Leserahmen vollständig war. Die Sequenz wies 74 % Identität mit der Sequenz aus Cladosporium herbarum auf.

### Beispiel 9

Der offene Leserahmen des Klons kodierend für die Mannit-Dehydrogenase aus Alternaria alternata wurde nun rekloniert in dem Expressionsvektor pHIS-paralleI2 [P. Sheffield et al. (1999), Overcoming expression and purification problems of RhoGDI using a family of "parallel" expression vectors. Protein Exp. Purif. 15, 34] unter Verwendung der Restriktionsschnittstellen Bam H I (N-terminal) und Xho I (C-terminal). Bei einer Expression in E. coli BL21 und nachfolgender Analyse der Genprodukte mit Hilfe von SDS-PAGE-Gelelektrophorese und Coomassie-Blue-Färbung zeigte sich eine stark exprimierte Proteinbande bei einem Molekulargewicht von etwa 30 kD. Dies entspricht etwa dem theoretisch zu erwartenden Molekulargewicht.

### Beispiel 10

Um das rekombinant hergestellte und am C-Terminus mit einem Poly-HIS-Fragment versehene Protein zu reinigen, wurde folgende Vorgehensweise gewählt:
Die E.coli-Zellen mit dem Expressionsvektor, die das Fremdprotein, die Mannit-Dehydrogenase aus Alternaria alternata exprimieren, wurden zunächst in üblicher Weise lysiert. Dabei wurde festgestellt, dass das rekombinant hergestellte Protein in unlöslicher Form vorlag. Die durch die Überexpression des Fremdproteins gebildeten inclusion bodies wurden zunächst in einem Puffer mit 6-molarem Harnstoff solubilisiert und anschließend über eine Nickel-Chelat-Säule affinitätschromatographisch gereinigt. Der Auftrag der rekombinant hergestellten Mannit-Dehydrogenase erfolgte in 6M Harnstoff-Puffer. Zur Elution wurde Imidazol-Puffer eingesetzt. Die proteinhaltigen Fraktionen wurden anschließend durch eine präparative SDS-PAGE-Gelelektrophorese weiter gereinigt und analysiert und dabei zeigte sich, dass das Allergen bereits nahezu vollständig zur Homogenität gereinigt war. Bei einer Färbung mit Coomassie-BB-R zeigte sich nur noch eine Bande mit einem Molekulargewicht von etwa 30 kD.

### Beispiel 11

Das gemäß Beispiel 10 hergestellte Protein wurde gelelektrophoresisch aufgetrennt und in einem IgE-Immunblot mit 28 Patientenseren getestet. Die 28 Seren stammten alle von Patienten, die positiv mit dem Rohextrakt von Alternaria alternata reagierten hatten und die auch sowohl im Hauttest wie auch im RAST vorgetestet waren. Bei 9 der getesteten Patientenseren war eine starke Bande im Immunblot sichtbar. Dies entspricht etwa 32 % der mit Alternaria alternata sensibilisierten Patienten.

## Patentansprüche

1. Impfstoff zur Desensibilisierung von Patienten gegen eine Schimmelpilzallergie, **dadurch gekennzeichnet, dass** er wenigstens ein Polypeptid mit wenigstens einem Epitop, das wenigstens 11 aufeinanderfolgende Aminosäuren aus der Aminosäuresequenz mit der Sequenz ID Nr. 1 oder der Sequenz ID Nr. 8 oder eine Sequenz ausgewählt aus Seq.ID Nr. 4, 5 und 6 aufweist oder das wenigstens 11 aufeinanderfolgende Aminosäuren aus der Aminosäuresequenz mit der Sequenz ID Nr. 11 aufweist, enthält.

2. Verwendung eines Polypeptids mit wenigstens einem Epitop, das wenigstens 11 aufeinanderfolgende Aminosäuren aus der Aminosäuresequenz mit der Sequenz ID Nr. 1 oder der Sequenz ID Nr. 8 oder eine Sequenz ausgewählt aus Seq.ID Nr. 4, 5 und 6 aufweist oder das wenigstens 11 aufeinanderfolgende Aminosäuren aus der Aminosäuresequenz mit der Sequenz ID Nr. 11 aufweist, zur Herstellung eines Impfstoffs gegen eine Schimmelpilzallergie.

3. Verwendung eines Polypeptids, das wenigstens 11 aufeinanderfolgende Aminosäuren aus der Aminosäuresequenz mit der Sequenz ID Nr. 1 oder der Sequenz ID Nr. 8 oder eine Sequenz ausgewählt aus Seq.ID Nr. 4, 5 und 6 aufweist oder das wenigstens 11 aufeinanderfolgende Aminosäuren aus der Aminosäuresequenz mit der Sequenz ID Nr. 11 aufweist, zum diagnostischen Nachweis einer Erkrankung, nämlich einer Allergie.

4. Verwendung nach Anspruch 3, **dadurch gekennzeichnet, dass** es sich bei der Erkrankung um eine Allergie gegen Schimmelpilze handelt.

## Claims

1. Vaccine for desensitizing patients to a mold allergy, **characterized in that** it comprises at least one polypeptide with at least one epitope which has at least 11 consecutive amino acids from the amino acid sequence with the sequence ID No. 1 or the sequence ID No. 8 or a sequence selected from among seq. ID No. 4, 5 and 6 or which has at least 11 consecutive amino acids from the amino acid sequence with the sequence ID No. 11.

2. Use of a polypeptide with at least one epitope which has at least 11 consecutive amino acids from the amino acid sequence with the sequence ID No. 1 or the sequence ID No. 8 or a sequence selected from among seq. ID No. 4, 5 and 6 or which has at least 11 consecutive amino acids from the amino acid sequence with the sequence ID No. 11 for the preparation of a vaccine for the treatment of mold allergy.

3. Use of a polypeptide which has at least 11 consecutive amino acids from the amino acid sequence with the sequence ID No. 1 or the sequence ID No. 8 or a sequence selected from among seq. ID No. 4, 5 and 6 or which has at least 11 consecutive amino acids from the amino acid sequence with the sequence ID No. 11 for the diagnostic detection of a disease, namely an allergy.

4. Use according to claim 3, **characterized in that** the disease is an allergy to mold.

## Revendications

1. Vaccin pour la désensibilisation de patients contre une allergie à une moisissure, **caractérisé en ce qu'**il contient au moins un polypeptide comportant au moins un épitope qui présente au moins 11 acides aminés consécutifs de la séquence d'acides aminés comportant la séquence ID n° : 1 ou la séquence ID n° 8 ou une séquence choisie parmi Seq. ID n° 4, 5 et 6 ou qui présente au moins 11 acides aminés consécutifs de la séquence d'acides aminés omportant la séquence ID n° 11.

2. Utilisation d'un polypeptide comportant au moins un épitope qui présente au moins 11 acides aminés consécutifs de la séquence d'acides comportant la séquence ID n° 1 ou la séquence ID n° 8 ou une séquence choisie parmi SEQ ID n° 4, 5 et 6 ou qui présente au moins 11 acides aminés consécutifs de la séquence d'acides aminés comportant la séquence ID n° : 11, pour la production d'un vaccin contre une allergie à une moisissure.

3. Utilisation d'un polypeptide qui présente au moins 11 acides aminés consécutifs de la séquence d'acides aminés comportant la séquence ID n° 1 ou la séquence ID n° 8 ou une séquence choisie parmi SEQ ID n° 4, 5 et 6 ou qui présente au moins 11 acides aminés consécutifs de la séquence d'acides aminés comportant la séquence ID n° : 11, pour la détection diagnostique d'une maladie, à savoir une allergie.

4. Utilisation selon la revendication 3, **caractérisée en ce que** la maladie est une allergie à une moisissure.


### SEQUENZPROTOKOLL

<110> Biomay Produktions- und Handels-Gesellschaft m.b.H
<120> Polypeptid kodierend für eine Mannit-Dehydrogenase, dessen Herstellung und Verwendung in Diagnostik und Therapie
<130> Cla h 1
<140>
   <141>
<150> 102 14 082.0
   <151> 2002-03-28
<150> 102 33 676.8
   <151> 2002-07-24
<160> 12
<170> PatentIn Ver. 2.1
<210> 1
   <211> 173
   <212> PRT
   <213> Cladosporium herbarum
<400> 1
   Leu Lys Gly Lys Val Val Val Val Thr Gly Ala Ser Gly Pro Lys Gly
     1               5                  10                  15
   Met Gly Ile Glu Ala Ala Arg Gly Cys Ala Glu Met Gly Ala Ala Val
                20                  25                  30
   Ala Ile Thr Tyr Ala Ser Arg Ala Gln Gly Ala Glu Glu Asn Val Lys
            35                  40                  45
   Glu Leu Glu Lys Thr Tyr Gly Ile Lys Ala Lys Ala Tyr Lys Cys Gln
        50                  55                  60
   Val Asp Ser Tyr Glu Ser Cys Glu Lys Leu Val Lys Asp Val Val Ala
    65                  70                  75                  80
   Asp Phe Gly Gln Ile Asp Ala Phe Ile Ala Asn Ala Gly Ala Thr Ala
                    85                  90                  95
   Asp Ser Gly Ile Leu Asp Gly Ser Val Glu Ala Trp Asn His Val Val
        100                 105                 110
   Gln Val Asp Leu Asn Gly Thr Phe His Cys Ala Lys Ala Val Gly His
           115                 120                 125
   His Phe Lys Glu Arg Gly Thr Gly Ser Phe Val Ile Thr Ser Ser Met
       130                 135                 140
   Ser Gly His Ile Ala Asn Tyr Pro Gln Glu Gln Thr Ser Tyr Asn Val
   145                 150                 155                 160
   Ala Lys Ala Gly Cys Ile His Met Ala Arg Ser Leu Ala
                   165                 170
<210> 2
   <211> 519
   <212> DNA
   <213> Cladosporium herbarum
<400> 2
   ctgaagggca aggtcgtcgt cgttaccggc gcttccggcc ccaagggcat gggtattgag 60
   gccgctcgcg gttgcgccga gatgggcgcc gctgttgcca tcacctacgc ctcccgcgcc 120
   cagggtgctg aggagaacgt caaggagctt gagaagacct acggcatcaa ggccaaggcc 180
   tacaagtgcc aggtcgacag ctacgagtcc tgcgagaagc tcgtcaagga cgtcgttgcc 240
   gacttcggcc agatcgatgc cttcatcgcc aacgccggtg ccaccgccga ctctggcatc 300
   ctcgacggct ccgtcgaggc ctggaaccac gtcgtccagg tcgacctgaa cggtaccttc 360
   cactgcgcca aggccgttgg ccaccacttc aaggagcgtg gaaccggttc cttcgtcatc 420
   acctcctcca tgtccggcca catcgccaac tatccccagg aacagacctc ctacaacgtc 480
   gccaaggctg gatgcatcca catggctcgc tccttggca                        519
<210> 3
   <211> 266
   <212> PRT
   <213> Cladosporium fulvum
<400> 3
<210> 4
   <211> 16
   <212> PRT
   <213> Cladosporium herbarum
<400> 4
   Pro Gly Gln Gln Ala Thr Lys His Glu Ser Leu Leu Asp Gln Leu Ser
   1 5 10 15
<210> 5
   <211> 11
   <212> PRT
   <213> Cladosporium herbarum
<400> 5
   Leu Asp Thr Gly Leu Ser Asp Phe Val Val Lys
   1 5 10
<210> 6
   <211> 10
   <212> PRT
   <213> Cladosporium herbarum
<400> 6
   Met Gly Arg Asp Gly Leu Ala Lys Glu Leu
   1 5 10
<210> 7
   <211> 918
   <212> DNA
   <213> Cladosporium herbarum
<400> 7
   ccgtctacac acgcaacttc ccgcctcgac tccatatcca atcacatcaa gatgcctggc 60
   cagcaagcaa ccaagcatga gtcccttttg gaccagctct ccctgaaggg caaggtcgtc 120
   gtcgtcaccg gcgcttccgg ccccaagggc atgggtattg aggccgctcg cggttgcgcc 180
   gagatgggcg ccgctgttgc catcacctac gcctcccgcg cccagggtgc tgaggagaac 240
   gtcaaggagc ttgagaagac ctacggcatc aaggccaagg cctacaagtg ccaggtcgac 300
   agctacgagt cctgcgagaa gctcgtcaag gacgtcgttg ccgacttcgg ccagatcgat 360
   gccttcatcg ccaacgccgg tgccaccgcc gactctggca tcctcgacgg ctccgtcgag 420
   gcctggaacc acgtcgtcca ggtcgacctg aacggtacct tccactgcgc caaggccgtt 480
   ggccaccact tcaaggagcg tggaaccggt tccctcgtca tcaccgcctc catgtccggc 540
   cacatcgcca acttccccca ggagcagacc tcctacaacg tcgccaaggc tggctgcatc 600
   cacatggctc gctccctcgc caacgagtgg cgcgacttcg cccgtgtcaa ctccatctcc 660
   cccggttaca ttgacactgg tctctccgac ttcgttccca aggagaccca gcagctctgg 720
   cactccatga tccccatggg ccgtgacggt ctcgccaagg agctcaaggg cgcctacgtc 780
   tacttcgcct ccgacgcctc cacctacacc accggtgccg atctcctcat tgacggtggt 840
   tacaccacca gataagcgac tcgcccacag caagtcgttg aggcggaagg acaaaaaaaa 900
   aaaaaaaaaa aaaaaaaa 918
<210> 8
   <211> 267
   <212> PRT
   <213> Cladosporium herbarum
<400> 8
   Met Pro Gly Gln Gln Ala Thr Lys His Glu Ser Leu Leu Asp Gln Leu
    1              5                   10                  15
   Ser Leu Lys Gly Lys Val Val Val Val Thr Gly Ala Ser Gly Pro Lys
               20                  25                  30
   Gly Met Gly Ile Glu Ala Ala Arg Gly Cys Ala Glu Met Gly Ala Ala
           35                  40                  45
   Val Ala Ile Thr Tyr Ala Ser Arg Ala Gln Gly Ala Glu Glu Asn Val
       50                  55                  60
   Lys Glu Leu Glu Lys Thr Tyr Gly Ile Lys Ala Lys Ala Tyr Lys Cys
   65                  70                  75                  80
   Gln Val Asp Ser Tyr Glu Ser Cys Glu Lys Leu Val Lys Asp Val Val
                   85                  90                  95
   Ala Asp Phe Gly Gln Ile Asp Ala Phe Ile Ala Asn Ala Gly Ala Thr
               100                 105                 110
   Ala Asp Ser Gly Ile Leu Asp Gly Ser Val Glu Ala Trp Asn His Val
           115                 120                 125
   Val Gln Val Asp Leu Asn Gly Thr Phe His Cys Ala Lys Ala Val Gly
       130                 135                 140
   His His Phe Lys Glu Arg Gly Thr Gly Ser Leu Val Ile Thr Ala Ser
   145                 150                 155                 160
   Met Ser Gly His Ile Ala Asn Phe Pro Gln Glu Gln Thr Ser Tyr Asn
                   165                 170                 175
   Val Ala Lys Ala Gly Cys Ile His Met Ala Arg Ser Leu Ala Asn Glu
               180                 185                 190
   Trp Arg Asp Phe Ala Arg Val Asn Ser Ile Ser Pro Gly Tyr Ile Asp
           195                 200                 205
   Thr Gly Leu Ser Asp Phe Val Pro Lys Glu Thr Gln Gln Leu Trp His
       210                 215                 220
   Ser Met Ile Pro Met Gly Arg Asp Gly Leu Ala Lys Glu Leu Lys Gly
   225                 230                 235                 240
   Ala Tyr Val Tyr Phe Ala Ser Asp Ala Ser Thr Tyr Thr Thr Gly Ala
                   245                 250                 255
   Asp Leu Leu Ile Asp Gly Gly Tyr Thr Thr Arg
               260                 265
<210> 9
   <211> 266
   <212> PRT
   <213> Cladosporium fulvum
<400> 9
   Met Pro Gln Arg Ile Pro Glu Ala Glu His Leu Leu Asp Leu Leu Ser
     1               5                  10                  15
   Leu Lys Gly Arg Val Val Val Val Thr Gly Ala Ser Gly Pro Lys Gly
                20                  25                  30
   Met Gly Ile Glu Ala Ala Arg Gly Cys Ala Glu Met Gly Ala Asp Leu
            35                  40                  45
   Ala Ile Thr Tyr Ala Ser Arg Ala Glu Gly Gly Leu Lys Asn Ala Glu
        50                  55                  60
   Glu Leu Ser Lys Gln Tyr Gly Ile Lys Cys Lys Ala Tyr Lys Cys Gln
    65                  70                  75                  80
   Val Asp Lys Tyr Glu Ser Val Glu Gln Leu Val Lys Asp Val Ile Gln
                    85                  90                  95
   Asp Phe Gly Lys Ile Asp Ala Phe Ile Ala Asn Ala Gly Ala Thr Ala
               100                 105                 110
   Asn Ser Gly Ile Leu Asp Gly Ser Val Glu Asp Trp Asn His Val Val
           115                 120                 125
   Gln Val Asp Leu Asn Gly Thr Phe His Cys Ala Lys Ala Val Gly His
       130                 135                 140
   His Phe Lys Glu Arg Gly Thr Gly Ser Phe Val Ile Thr Ser Ser Met
   145                 150                 155                 160
   Ser Gly His Ile Ala Asn Tyr Pro Gln Glu Gln Thr Ser Tyr Asn Val
                   165                 170                 175
   Ala Lys Ala Gly Cys Ile His Met Ala Arg Ser Leu Ala Asn Glu Trp
               180                 185                     190
   Arg Asp Phe Ala Arg Val Asn Ser Ile Ser Pro Gly Tyr Ile Asp Thr
           195                 200                     205
   Gly Leu Ser Asp Phe Val Ala Lys Asp Ile Gln Lys Leu Trp His Ser
       210                 215                     220
   Met Ile Pro Leu Gly Arg Asp Gly Leu Ala Lys Glu Leu Lys Gly Ala
   225                 230                     235                 240
   Tyr Val Tyr Leu Val Ser Asp Ala Ser Thr Tyr Thr Thr Gly Ala Asp
                   245                 250                     255
   Ile Val Ile Asp Gly Gly Tyr Thr Cys Arg
               260                 265
<210> 10
   <211> 267
   <212> PRT
   <213> Cladosporium herbarum
<400> 10
   Met Pro Gly Gln Gln Ala Thr Lys His Glu Ser Leu Leu Asp Gln Leu
    1               5                  10                  15
   Ser Leu Lys Gly Lys Val Val Val Val Thr Gly Ala Ser Gly Pro Lys
               20                  25                  30
   Gly Met Gly Ile Glu Ala Ala Arg Gly Cys Ala Glu Met Gly Ala Ala
           35                  40                  45
   Val Ala Ile Thr Tyr Ala Ser Arg Ala Gln Gly Ala Glu Glu Asn Val
       50                  55                  60
   Lys Glu Leu Glu Lys Thr Tyr Gly Ile Lys Ala Lys Ala Tyr Lys Cys
   65                  70                  75                  80
   Gln Val Asp Ser Tyr Glu Ser Cys Glu Lys Leu Val Lys Asp Val Val
                   85                  90                  95
   Ala Asp Phe Gly Gln Ile Asp Ala Phe Ile Ala Asn Ala Gly Ala Thr
               100                 105                 110
   Ala Asp Ser Gly Ile Leu Asp Gly Ser Val Glu Ala Trp Asn His Val
           115                 120                 125
   Val Gln Val Asp Leu Asn Gly Thr Phe His Cys Ala Lys Ala Val Gly
       130                 135                 140
   His His Phe Lys Glu Arg Gly Thr Gly Ser Leu Val Ile Thr Ala Ser
   145                 150                 155                 160
   Met Ser Gly His Ile Ala Asn Phe Pro Gln Glu Gln Thr Ser Tyr Asn
                   165                 170                 175
   Val Ala Lys Ala Gly Cys Ile His Met Ala Arg Ser Leu Ala Asn Glu
               180                 185                 190
   Trp Arg Asp Phe Ala Arg Val Asn Ser Ile Ser Pro Gly Tyr Ile Asp
           195                 200                 205
   Thr Gly Leu Ser Asp Phe Val Pro Lys Glu Thr Gln Gln Leu Trp His
       210                 215                 220
   Ser Met Ile Pro Met Gly Arg Asp Gly Leu Ala Lys Glu Leu Lys Gly
   225                 230                 235                 240
   Ala Tyr Val Tyr Phe Ala Ser Asp Ala Ser Thr Tyr Thr Thr Gly Ala
                   245                 250                 255
   Asp Leu Leu Ile Asp Gly Gly Tyr Thr Thr Arg
               260                 265
<210> 11
   <211> 266
   <212> PRT
   <213> Alternaria alternata
<400> 11
   Met Pro Ile Thr Val Pro Gln Ala Thr Glu Leu Lys Asp Leu Phe Ser
    1               5                  10                  15
   Leu Lys Gly Lys Val Val Ile Val Thr Gly Ala Ser Gly Pro Thr Gly
               20                  25                  30
   Ile Gly Thr Glu Ala Ala Arg Gly Cys Ala Glu Tyr Gly Ala Asp Leu
           35                  40                  45
   Ala Ile Thr Tyr Asn Ser Arg Ala Glu Gly Ala Glu Lys Asn Ala Lys
       50                  55                  60
   Glu Met Ser Glu Lys Tyr Gly Val Lys Val Lys Ala Tyr Lys Cys Gln
    65                 70                  75                  80
   Val Asn Glu Tyr Ala Gln Cys Glu Lys Leu Val Gln Asp Val Ile Lys
                   85                  90                  95
   Asp Phe Gly Lys Val Asp Val Phe Ile Ala Asn Ala Gly Lys Thr Ala
               100                 105                 110
   Asp Asn Gly Ile Leu Asp Ala Thr Val Glu Gln Trp Asn Glu Val Ile
           115                 120                 125
   Gln Thr Asp Leu Thr Gly Thr Phe Asn Cys Ala Arg Ala Val Gly Leu
       130                 135                 140
   His Phe Arg Glu Arg Lys Thr Gly Ser Leu Val Ile Thr Ser Ser Met
   145                 150                 155                 160
   Ser Gly His Ile Ala Asn Phe Pro Gln Glu Gln Ala Ser Tyr Asn Val
                   165                 170                 175
   Ala Lys Ala Gly Cys Ile His Leu Ala Lys Ser Leu Ala Asn Glu Trp
               180                 185                 190
   Arg Asp Phe Ala Arg Val Asn Ser Ile Ser Pro Gly Tyr Ile Asp Thr
           195                 200                 205
   Gly Leu Ser Asp Phe Val Pro Gln Asp Ile Gln Lys Leu Trp His Ser
       210                 215                 220
   Met Ile Pro Met Gly Arg Asp Ala Lys Ala Thr Glu Leu Lys Gly Ala
   225                 230                 235                 240
   Tyr Val Tyr Phe Ala Ser Asp Ala Ser Ser Tyr Cys Thr Gly Ser Asp
                   245                 250                 255
   Leu Leu Ile Asp Gly Gly Tyr Cys Val Arg
               260                 265
<210> 12
   <211> 1026
   <212> DNA
   <213> Alternaria alternata
<400> 12
   cttcatatca catcacactt caactcaatt cccattttat ataccccaaa cttctttact 60
   cttcataaac ccacataatc gccacaatgc ccatcaccgt tccccaagct accgagctca 120
   aggacctctt cagccttaag ggcaaggtcg tcatcgtcac cggtgcctcc ggccccaccg 180
   gtattggcac agaggctgcc cgaggatgcg ctgagtacgg tgccgacctc gccatcacct 240
   acaactctcg cgccgagggt gccgagaaga acgcaaagga gatgagcgag aagtacggcg 300
   tcaaggtcaa ggcctacaag tgccaggtca acgagtacgc tcagtgcgag aagctcgtcc 360
   aggacgtcat caaggacttc ggcaaggtcg atgtcttcat cgccaacgcc ggaaagactg 420
   ccgacaacgg tatcctcgac gctaccgttg agcagtggaa cgaggtcatc cagaccgact 480
   tgaccggtac cttcaactgc gcccgtgccg ttggtctcca cttccgcgag cgcaagactg 540
   gctctctcgt catcacctcc tccatgtccg gccacattgc caacttcccc caggagcagg 600
   cctcctacaa cgttgctaag gctggctgca ttcacctcgc caagtcgctc gccaacgagt 660
   ggagggactt tgcccgtgtc aactccatct cccctggata cattgacact ggtctctccg 720
   acttcgttcc ccaggacatc cagaagctgt ggcactccat gatccccatg ggccgtgacg 780
   ccaaggctac tgagctcaag ggtgcctacg tctacttcgc atcggatgcc tcatcctact 840
   gcactggttc cgatctcctc atcgacggtg gttactgcgt caggtaaacg tgtcattccg 900
   gaaggaagat gcgagtggag gaatataata atggacgacg tcttgccgga agtcttgtgt 960
   ccatgtaaat agcatcgaga catcaataaa gcttcgcagg tttcacatca caaaaaaaaa 1020
   aaaaaa                                                            1026